Europäisches Patentamt

European Patent Office

Office européen des brevets

(19)

(11) Numéro de publication: **0 130 930**
**A2**

(12) # DEMANDE DE BREVET EUROPEEN

(21) Numéro de dépôt: 84450019.9

(22) Date de dépôt: **29.06.84**

(51) Int. Cl.⁴: **A 61 M 16/00**

(30) Priorité: **04.07.83 FR 8311212**

(43) Date de publication de la demande: **09.01.85**
**Bulletin 85/2**

(84) Etats contractants désignés: **AT BE CH DE FR GB IT LI LU NL SE**

(71) Demandeur: **Desgranges, Albert, 11 Rue des Anoubles, F-34000 Montpellier (FR)**

(72) Inventeur: **Desgranges, Albert, 11 Rue des Anoubles, F-34000 Montpellier (FR)**

(74) Mandataire: **Ravina, Bernard, Cabinet Bernard RAVINA 24, boulevard Riquet, F-31000 Toulouse (FR)**

(54) **Procédé de mesure des pressions endotrachéales dans la respiration artificielle par injection de gaz à haute fréquence et appareil de respiration artificielle mettant en oeuvre le dit procédé.**

(57) Procédé de mesure des pressions endotrachéales dans la respiration artificielle par injection de gaz à haute fréquence et appareil de respiration artificielle mettant en œuvre ledit procédé.

L'invention concerne un procédé de mesure des pressions endotrachéales dans la respiration artificielle par injection de gaz à haute fréquence dans les voies respiratoires de l'homme ou de l'animal et un appareil mettant en œuvre ledit procédé.

Le procédé selon l'invention se caractérise en ce que la mesure par un dispositif adapté (6), (7), (8) s'effectue à travers le tube (5) d'injection des gaz pendant la phase expiratoire et après la détente des gaz contenus dans le circuit d'injection, c'est-à-dire dans le tube (5) qui est doté d'un dispositif d'échappement (4) au niveau de l'extrémité proximale dudit tube.

0130930

1

PROCEDE DE MESURE DES PRESSIONS ENDOTRACHEALES DANS LA RESPIRATION ARTIFICIELLE PAR INJECTION DE GAZ A HAUTE FREQUENCE ET APPAREIL DE RESPIRATION ARTIFICIELLE METTANT EN OEUVRE LE DIT PROCEDE.

L'invention concerne un procédé de mesure des pressions endotracheales dans la respiration artificielle par injection de gaz à haute fréquence

L'invention concerne également un appareil de respiration artificielle procédant par injection de gaz à haute fréquence.

La ventilation par injection de gaz ou "jet ventilation" des Anglosaxons à fréquence élevée est une technique de ventilation artificielle en pression positive intermittente déterminée par l'injection de gaz ou d'un mélange de gaz sous pression à travers une sonde de petit calibre, appelé injecteur, qui est introduit dans la trachée de l'homme (ou de l'animal) soit à travers une sonde d'intubation endotracheale ou de tracheotomie, soit directement par voie orale ou nasale soit par voie cervicale transcutanée.

On connaît déjà des appareils de respiration artificielle par injection de gaz à haute fréquence (Respiratory apparatus UK Patent application N° 2.025.240A).

Ils fonctionnent tous selon le même principe qui est le découpage d'un flux de gaz à haute pression (1 à 5 bars) au moyen d'une électrovanne ou d'un autre dispositif, qui règle l'alternance des périodes d'insufflation et d'expiration.

Le flux gazeux, après découpage, est injecté dans la trachée de l'homme ou de l'animal à travers une sonde de petit calibre.

La sonde peut être introduite dans la trachée soit par voie orale, soit par voie cervicale transcutanée.

0130930

Certains appareils tels que celui décrit dans le brevet mentionné plus haut sont pourvus d'un dispositif de surveillance et de sécurité dont le fonctionnement est basé sur la mesure des pressions endotrachéales.

Ces dispositifs posent le problème de la prise des pressions.

En effet, dans la respiration artificielle à haute fréquence, les pressions d'injection des gaz sont élevées, 1 à 5 bars, et les pressions endotrachéales sont faibles, 0 à 80 mbars, ce qui ne permet pas une mesure directe des pressions endotrachéales à travers le tuyau d'injection des gaz d'insufflation comme c'est le cas dans la respiration artificielle classique à basse fréquence.

Pour cette raison, les constructeurs utilisent un circuit pour la mesure des pressions qui est distinct du circuit d'injection des gaz.

Une telle technique oblige à utiliser et à mettre en place dans la sonde d'intubation endotrachéale ou de trachéotomie ou directement dans la trachée du patient deux tubes, l'un pour l'injection des gaz et l'autre pour la mesure des pressions, ou un tube unique à deux lumières mais d'un diamètre plus important.

Dans de nombreuses indications un tel dispositif n'est pas .utilisable ou souhaitable.

Le problème posé est celui de la mesure d'une pression basse, par exemple 0 à 80 mbars et de l'injection de gaz à haute pression par exemple de 1 à 5 bars, par un même tube endotrachéal de diamètre réduit et du fonctionnement des dispositifs d'alarme et de sécurité à partir des pressions mesurées.

La solution proposée par l'inventeur au problème précédent est la mesure des pressions endotrachéales à travers le même tube que celui qui sert à l'injection des gaz, la mesure étant selon l'invention effectuée en dehors des périodes d'injection des gaz et après un temps nécessaire à la détente des gaz contenus dans le circuit d'injection.

La détente des gaz après la fin de l'insufflation, donc au début de l'expiration s'effectue normalement par l'extrémité distale de l'injecteur, elle peut être accélérée par un échappement au niveau de l'extrémité proximale du circuit d'injection.

Par extrémité distale de l'injection, on entend l'extrémité la plus éloignée de l'injecteur par rapport à l'alimentation en gaz.

Par extrémité proximale de l'injection, on entend l'extrémité la plus proche du circuit d'injection par rapport à l'alimentation en gaz.

Cet échappement proximal permet d'avoir un temps de détente des gaz de très courte durée et relativement indépendant du diamètre de l'extrémité distale de l'injecteur.

Cet échappement réalise un système de décompression qui permet d'effectuer la mesure pendant la phase expiratoire et qui permet en outre d'obtenir en fin d'expiration un front descendant des pressions d'injection plus rapide que sans décompression.

Les pressions mesurées sont les pressions endotrachéales expiratoires.

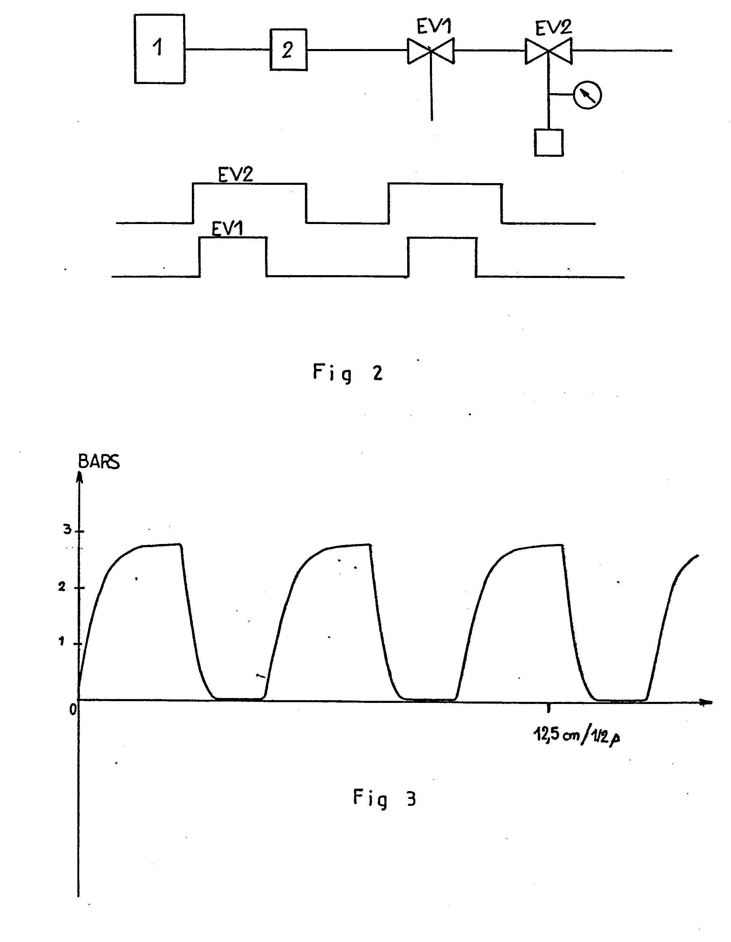

La mesure des pressions expiratoires ne permet pas de détecter directement les augmentations des pressions endotrachéales d'insufflation (fig.3).

Pour pallier cet inconvénient, l'inventeur propose un procédé et un dispositif basé sur la mesure de la pression de fin d'expiration,

4

l'appareil selon une caractéristique de l'invention n'injecte les gaz que si la pression endotrachéale, avant l'injection donc en fin d'expiration descend en dessous d'un seuil de sécurité réglé par l'opérateur.

Ce dispositif, qui est destiné à prévenir les accidents dus à l'augmentation des pressions d'insufflation diffère fondamentalement de ceux qui existent sur les appareils de respiration artificielle actuels qui arrêtent l'insufflation lorsque la pression d'insufflation atteint une valeur maxima réglée par l'opérateur.

Les avantages obtenus grâce à cette invention consistent essentiellement en ce que les pressions endotrachéales sont mesurées à travers le même tuyau que celui qui sert à l'injection des gaz sous haute pression.

Ce qui permet d'utiliser ce type de ventilation avec des sécurités et des alarmes sur les pressions dans toutes les indications où un seul tube endotrachéal est utilisable ou souhaitable.

D'autres avantages et caractéristiques de l'invention apparaîtront à la lecture de la description ci-après d'une forme de réalisation de l'invention donnée à titre d'exemple non limitatif et illustrée par les dessins joints dans lesquels :

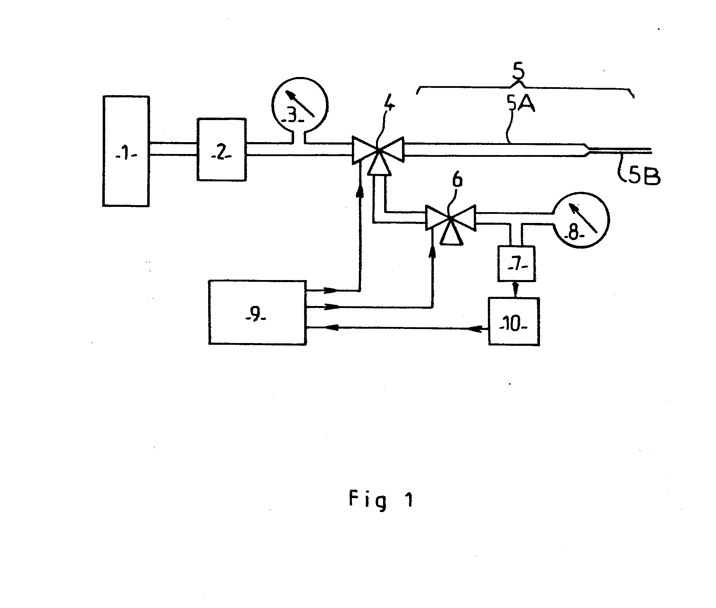

- la figure 1 est une représentation schématique d'une forme de réalisation,
- la figure 2 est une représentation schématique d'une autre forme de réalisation de l'invention,
- la figure 3 est une représentation du front descendant des pressions dans le circuit patient en phase d'expiration.

Le respirateur est alimenté par des gaz sous pression 1. Un détendeur 2 permet de régler la pression de travail du respirateur, cette
pression est lue sur un manomètre 3.

Une électrovanne à trois voies 4 ou un dispositif équivalent disposée au niveau de l'extrémité proximale du circuit d'injection commande d'une part l'admission des gaz dans le circuit patient 5 pendant la phase d'insufflation et d'autre part l'arrêt des gaz et le
branchement du circuit patient sur le circuit de mesure des pressions pendant la phase d'expiration.

Cette électrovanne constitue le moyen d'échappement.


Le circuit d'injection 5 ou circuit patient est constitué par le
tube d'injection 5A et l'injecteur 5B.

Pour simplifier la description, il sera fait référence de manière
générique au circuit d'injection 5.

Le circuit de mesure des pressions comprend, une électrovanne 6 à
trois voies ou un dispositif équivalent, qui permet pendant la phase
d'expiration et par l'intermédiaire de l'électrovanne 4 dans un premier temps d'accélérer la détente des gaz contenus dans le circuit
patient par échappement des gaz à l'extérieur du circuit et dans un
deuxième temps de connecter le circuit patient avec un capteur de
pression 7 et un manomètre de contrôle 8 pour la mesure des pressions endotrachéales.

Le cycle d'ouverture et de fermeture des deux électrovannes est
assuré par un dispositif de commande électronique 9.


Les informations recueillies par le capteur de pression sont utilisés pour le fonctionnement du dispositif de sécurtié et d'alarme 10.

Le dispositif de sécurité et d'alarme compare la pression mesurée en
fin d'expiration à deux valeurs l'une basse et l'autre haute, rég-

0130930

lées par l'opérateur en fonction des pressions ventilatoires du patient de telle façon que la pression mesurée en fin d'expiration oscille normalement entre ces deux valeurs.

Une baisse de la pression expiratoire du patient en dessous de la pression de sécurité basse entraîne le déclenchement d'une alarme sonore et visuelle pour signaler une baisse anormale de pression. Une augmentation de la pression de fin d'expiration au dessus de la pression de sécurité haute entraînera le déclenchement d'une alarme sonore et visuelle et le blocage, par l'intermédiaire du système de commande, de l'injection des gaz jusqu'à ce que la pression de fin d'expiration descende en dessous de la pression de sécurité haute.

En figure 2 est représentée une autre forme de réalisation de l'invention avec électrovanne EV1 d'échappement et électrovanne EV2 reliée au capteur et illstration des séquences de fonctionnement.

Le respirateur artificiel objet de l'invention trouve son utilisation dans toutes les indications de la respiration artificielle par injection de gaz à haute fréquence et plus particulièrement dans tous les cas ou un seul tube endotrachéal peut être utilisé.

REVENDICATIONS :          7

1. Procédé de mesure des pressions endotrachéales dans le cadre de la respiration artificielle par injection de gaz à pression élevée dans les voies respiratoires de l'homme ou de l'animal au moyen d'un circuit d'injection (5) caractérisé en ce que la mesure par un dispositif adapté (6),(7) (8) s'effectue à travers le tube (5) d'injection des gaz pendant la phase expiratoire et après la détente des gaz contenus dans le circuit d'injection.

2. Procédé de mesure des pressions endotrachéales dans le cadre de la respiration artificielle par injection de gaz à pression élevée selon la revendication 1 caractérisé par la réalisation d'un échappement des gaz et de décompression entre l'extrémité proximale et l'extrémité distale du circuit d'injection (5).

3. Appareil de respiration artificielle procédant par injection des gaz à pression élevée dans les voies respiratoires de l'homme ou de l'animal et mettant en oeuvre le procédé selon la revendication 1, le dit appareil étant caractérisé par un dispositif (6),(7),(8) de mesure des pressions endotrachéales à travers le tube d'injection des gaz (5) en relation avec lui pendant la phase expiratoire et après la détente des gaz contenus dans le circuit d'injection.

4. Appareil selon la revendication 2 caractérisé par un dispositif (4) d'échappement des gaz et de décompression au niveau de l'extrémité proximale du circuit (5) d'injection des gaz pour accelérer la détente des gaz dans le circuit d'injection au début de la phase expiratoire.

8

5. Appareil selon les revendications 2 et 3 caractérisé en ce que le dispositif d'échappement 4 est en relation avec le dispositif de mesure.

6. Appareil selon la revendication 2 caractérisé en ce que le dispositif d'échappement est une vanne à trois voies reliée à l'alimentation et au tube d'injection (5) et qui interrompt cette liaison pour relier le tube d'injection avec le dispositif de mesure ou avec l'air libre.

7. Appareil selon les revendications 2, 3, 4 et 5 caractérisé en ce que le dispositif de mesure (6),(7),(8) est relié à un dispositif de sécurité qui bloque l'injection des gaz et déclenche une alarme lorsque la pression endotrachéale expiratoire du sujet ventilé ne descend pas en dessous d'un seuil de sécurité réglé par l'opérateur qui assure le réglage et la mise en fonction de l'appareil de respiration.

Fig 1

0130930

Fig 2

Fig 3